(19) Europäisches Patentamt
European Patent Office
Office européen des brevets

(11) Veröffentlichungsnummer: **0 230 239**
**A2**

(12) # EUROPÄISCHE PATENTANMELDUNG

(21) Anmeldenummer: **87100242.4**

(22) Anmeldetag: **10.01.87**

(51) Int. Cl.[4]: **C07D 471/04**, A01N 43/90, //(C07D471/04,221:00,221:00)

(30) Priorität: **17.01.86 DE 3601688**

(43) Veröffentlichungstag der Anmeldung: **29.07.87 Patentblatt 87/31**

(84) Benannte Vertragsstaaten: **AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Nordhoff, Erhard, Dr.**
**Markelstrasse 45**
**D-1000 Berlin 41(DE)**
Erfinder: **Franke, Wilfried, Dr.**
**Karl-Stieler-Strasse 2 b**
**D-1000 Berlin 41(DE)**
Erfinder: **Arndt, Friedrich, Dr.**
**Mühlenfeldstrasse 10**
**D-1000 Berlin 28(DE)**
Erfinder: **Herrmann, Christoph, Dr.**
**Matterhornstrasse 76 g**
**D-1000 Berlin 38(DE)**
Erfinder: **Kötter, Clemens, Dr.**
**Laurinsteig 26**
**D-1000 Berlin 28(DE)**

(54) **Naphthyridin-Derivate, Verfahren zu ihrer Herstellung und ihre Verwendung als Mittel mit herbizider Wirkung.**

(57) Es werden neue Imidazolinyl-naphthyridin-Derivate der allgemeinen Formel

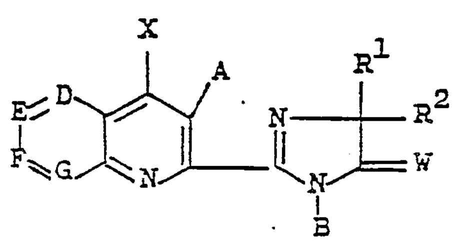

,

in der eines der Symbole D, E, F oder G ein Stickstoffatom und die anderen Symbole ein mit Y substituiertes Kohlenstoff-Atom bedeuten, wobei Y, X, A, B, $R^1$ und $R^2$ die in der Beschreibung angegebene Bedeutung haben, und ihre Herstellung beschrieben. Die erfindungsgemäßen Verbindungen sind als Herbizide verwendbar und zeichnen sich durch eine hohe Selektivität aus.

EP 0 230 239 A2

## Naphthyridin-Derivate, Verfahren zu ihrer Herstellung und ihre Verwendung als Mittel mit herbizider Wirkung

Die Erfindung betrifft neue Imidazolinyl-naphthyridin-Derivate, Verfahren zu ihrer Herstellung und ihre Verwendung als Mittel mit herbizider Wirkung.

Imidazoline mit herbiziden Eigenschaften sind bereits bekannt (DE OS 28 33 274 und 32 21 736). Die Wirkung dieser Verbindungen und die Verträglichkeit für landwirtschaftliche Kulturen ist nicht immer völlig zufriedenstellend bzw. es treten bei entsprechender Herbizidwirkung Selektivitätsprobleme in landwirtschaftlichen Hauptkulturen auf.

Die Aufgabe der vorliegenden Erfindung war es, Verbindungen bereitzustellen, die diese Nachteile nicht aufweisen und in ihren biologischen Eigenschaften den bekannten überlegen sind.

Es wurde nun gefunden, daß Naphthyridin-Derivate der allgemeinen Formel I

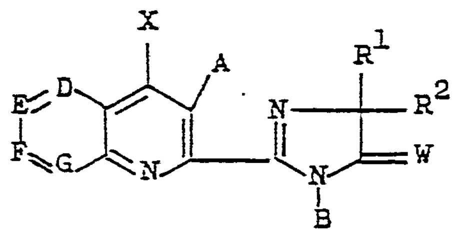

(I),

in der eines der Symbole D, E, F oder G ein Stickstoffatom und die anderen Symbole ein mit Y substituiertes Kohlenstoff-Atom bedeuten, wobei

Y für Wasserstoff, Halogen, Amino, Nitro, Cyano, $C_{3-8}$-Cycloalkyl, $C_{3-8}$-Cycloalkenyl, $C_{3-8}$-Cycloalkyloxy, $C_{3-8}$-Cycloalkenyloxy, $C_{1-8}$-Alkoxy, $C_{1-8}$-Alkoxycarbonyl, $C_{1-8}$-Alkylthio, $C_{3-8}$-Cycloalkylthio, Benzylthio, Tetrahydrofurfuryloxy; für freies oder mit Halogen, $C_{1-4}$-Alkyl oder $C_{1-4}$-Alkoxy substituiertes Phenyl; für freies oder mit $C_{1-4}$-Alkyl, Halogenalkyl oder Nitro substituiertes Phenoxy; für $C_{1-4}$-Alkylamino, Di-$C_{1-4}$-alkylamino, $C_{3-6}$-Cycloalkylamino; oder für einen ein-oder mehrfach mit Halogen, Cyano, Nitro, Amino oder $C_{1-4}$-Alkoxy substituierten $C_{1-8}$-Alkyl-, $C_{3-8}$-Alkenyl-oder $C_{3-8}$-Alkinylrest steht,

X für Wasserstoff, Nitro, $C_{1-3}$-Alkoxy, $C_{1-3}$ -Halogenalkyl und $C_{1-3}$-Alkyl steht,

$R^1$ für $C_{1-4}$-Alkyl steht,

$R^2$ für $C_{1-6}$-Alkyl oder $C_{3-6}$-Cycloalkyl steht, wobei

$R^1$ und $R^2$ gemeinsam einen gegebenenfalls mit Methyl substituierten $C_{3-6}$-Cycloalkylrest bedeuten können,

W für O oder S steht,

A für $CH_2OH$, $COOR^3$ oder $CONR^4R^5$ steht, wobei $R^3$ Wasserstoff, $C_{1-12}$-Alkyl, das durch ein oder mehrere O oder S unterbrochen sein kann und substituiert sein kann mit $C_{1-3}$-Alkoxy, Halogen, Hydroxy, $C_{3-6}$ -Cycloalkyl, Furyl, Tetrahydrofuryl, Phenyl, Halogenphenyl, $C_{1-4}$-Alkylphenyl, $C_{1-4}$-Alkoxyphenyl, Nitrophenyl, Cyano, Carboxyl, $C_{1-4}$-Alkoxycarbonyl oder $C_{1-4}$ -Alkylthio; $C_{3-8}$-Alkenyl, das mit $C_{1-3}$-Alkoxy, Phenyl oder Halogen substituiert sein kann; $C_{3-8}$-Alkinyl, das mit $C_{1-3}$-Alkoxy, Phenyl oder Halogen substituiert sein kann; $C_{3-6}$-Cycloalkyl, gegebenenfalls substituiert mit Methyl; oder ein Kation aus der Gruppe der Alkalimetalle, Erdalkalimetalle, Mangan, Kupfer, Eisen, Zink, Ammonium und organischen Ammoniumverbindungen bedeutet, und

$R^4$ und $R^5$ unabhängig voneinander für Wasserstoff, Hydroxy und $C_{1-4}$-Alkyl stehen, und falls $R^4$ Wasserstoff bedeutet, $R^5$ auch für $NH_2$, $N(CH_3)_2$, $NHCOCH_3$ und NH-Phenyl steht,

und

B für Wasserstoff steht und falls A $COOR^3$ ($R^3 \neq$ Wasserstoff) bedeutet, B auch $COR^6$ oder $SO_2R^7$ bedeutet, wobei $R^6$ für $C_{1-8}$-Alkyl, $C_{1-6}$-Halogenalkyl oder Phenyl steht, das mit Halogen, Nitro, Methyl oder Methoxy substituiert sein kann, und $R^7$ für $C_{1-6}$-Alkyl, $CF_3$, $CCl_3$ oder für ein gegebenenfalls mit Halogen, Nitro oder $C_{1-6}$-Alkyl substituiertes Phenyl steht, überraschenderweise eine größere Wirksamkeit bzw. eine verbesserte Selektivität aufweisen.

Mit dem Begriff Halogen ist Fluor, Chlor und Brom, vorzugsweise Fluor und Chlor gemeint.

Die erfindungsgemäßen Verbindungen der Formel I lassen sich nach folgenden Verfahren herstellen, indem man

A) Verbindungen der allgemeinen Formel II

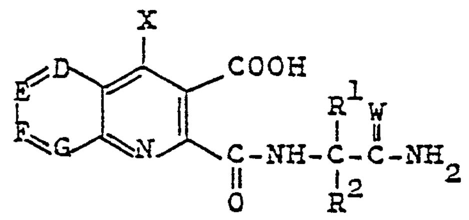

(II),

in der X, D, E, F, G, $R^1$, $R^2$ und W die in Formel I angegebene Bedeutung haben, alkalisch cyclisiert, wobei Verbindungen der Formel I erhalten werden, in der A = COOH und B = Wasserstoff bedeuten;

B) Verbindungen der allgemeinen Formel III

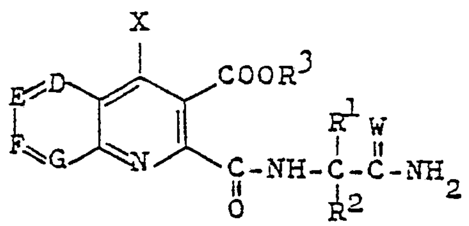

(III),

in der X, D, E, F, G, $R^1$, $R^2$, $R^3$ (jedoch nicht Wasserstoff) und W die in Formel I angegebene Bedeutung haben, mit einem Gemisch aus Phosphoroxichlorid/Phosphorpentachlorid umsetzt, wobei Verbindungen der Formel I erhalten werden in der A = $COOR^3$ ($R^3 \neq H$) und B = Wasserstoff bedeuten;

C) Verbindungen der allgemeinen Formel IV

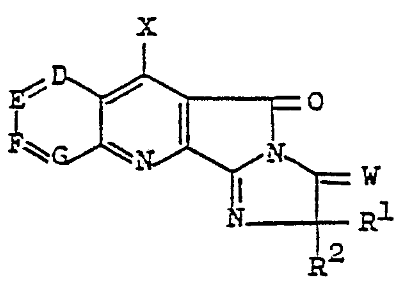

(IV),

in der, X, D, E, F, G, W, $R^1$ und $R^2$ die in Formel I angegebene Bedeutung haben,

C1) entweder mit einem Alkohol der Formel $R^3$-OH, wobei $R^3$ die in Formel I angegebene Bedeutung hat, jedoch nicht Wasserstoff, und dem entsprechenden Alkalimetallalkoxid, gegebenenfalls in Gegenwart eines Lösungsmittels umsetzt, oder

C2) mit einem Amin der Formel $HNR^4R^5$, wobei $R^4$ und $R^5$ die in Formel I angegebene Bedeutung haben, gegebenenfalls unter Verwendung eines Lösungsmittels umsetzt, oder

C3) mit einem Reduktionsmittel, vorzugsweise Natriumborhydrid, zur Reaktion bringt,

wobei Verbindungen der allgemeinen Formel I erhalten werden, in der A = $COOR^3$ ($R^3 \neq H$), $CONR^4R^5$ oder $CH_2OH$ und B = Wasserstoff bedeuten;

und gegebenenfalls eine Verbindung der Formel I, in der A für COOH steht, mit 1 Äquivalent einer Base als Salzbildner umsetzt, wobei Verbindungen der Formel I erhalten werden, in denen A für $COOR^3$ steht und $R^3$ ein salzbildendes Kation bedeutet,

oder

gegebenenfalls eine Verbindung der Formel I, in der A für $CO_2R^3$ ($R^3 \neq H$) steht, mit einem Acylhalogenid, Acylanhydrid oder Sulfonylhalogenid umsetzt, wobei Verbindungen der Formel I erhalten werden, in denen B für $COR^6$ oder $SO_2R^7$ steht.

Die Verfahrensvariante A) wird zweckmäßigerweise so durchgeführt, daß man das Ausgangsmaterial der Formel II einige Zeit im Bereich von 0,5 bis 20 Stunden bei einer Temperatur im Bereich von 20 bis 100 °C mit einer wäßrigen oder auch wäßrig-alkoholischen Alkalimetallhydroxid-Lösung, wie Natrium-oder Kaliumhydroxid-Lösung behandelt, wobei die Menge des Hydroxids, bezogen auf das Ausgangsmaterial der Formel II, 2-10 Moläquivalente beträgt. Anschließend wird das Reaktionsgemisch abgekühlt und mit einer starken Mineralsäure wie Schwefel-oder Salzsäure angesäuert.

Die als Ausgangsmaterial verwendeten Amide der Formel II lassen sich entsprechend dem folgenden Formelschema

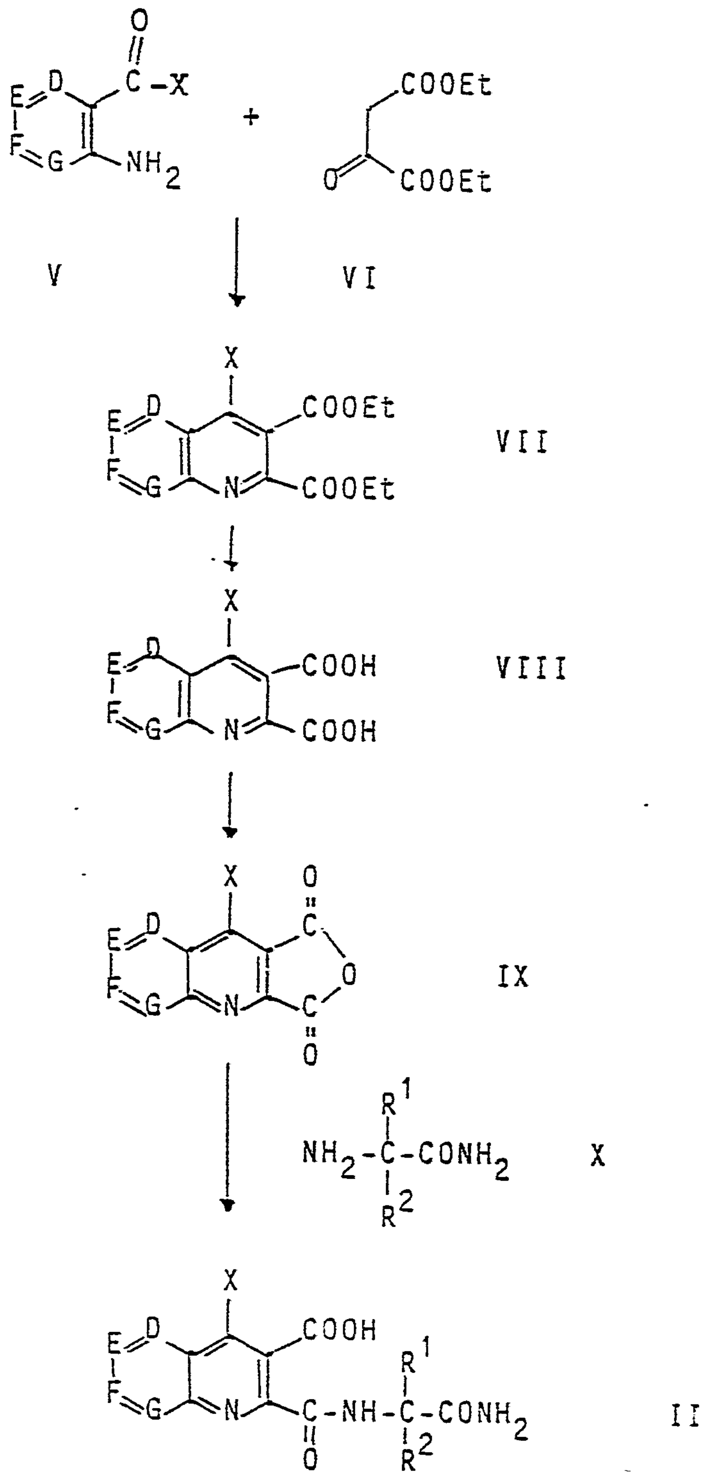

V

VI

VII

VIII

IX

X

II

herstellen, z.B. indem man Verbindungen der Formel V, in der D, E, F, G und X die in Formel I angegebene Bedeutung haben, mit Oxalessigsäurediethylester VI in die Naphthyridindicarbonsäurediethylester der Formel VII überführt. Die Diester der Formel VII lassen sich durch Behandlung mit einer starken Base wie Kaliumhydroxid oder Natriumhydroxid zu den Naphthyridindicarbonsäuren der Formel VIII hydrolysieren.

Die Säureanhydride der Formel IX werden durch Behandlung der Naphthyridindicarbonsäuren der Formel VIII mit beispielsweise Essigsäureanhydrid erhalten.

Durch Umsetzung mit einem Aminosäureamid der Formel X, in der $R^1$ und $R^2$ die in Formel I angegebene Bedeutung haben, werden Verbindungen der Formel II erhalten.

Die Herstellung von Verbindungen der Formel II erfolgt in Gegenwart eines organischen Lösungsmittels, wie z.B. Diethylether, Tetrahydrofuran, Acetonitril, Essigester oder in einem halogenierten Lösungsmittel, wie z.B. Methylenchlorid oder Chloroform. Die Reaktion findet in einem Temperaturbereich von 20 ° bis 100 °C statt. Die bei der Reaktion in geringen Mengen anfallenden isomeren Naphthyridincarbonsäurederivate lassen sich leicht durch Umkristallisation oder Chromatographie entfernen.

Die Verfahrensvarianten B) wird zweckmäßigerweise so durchgeführt, daß man das Ausgangsmaterial der Formel III direkt mit Phosphorpentachlorid in Phosphoroxichlorid bei Raumtemperatur zur Reaktion bringt. Die Reaktion kann auch in Gegenwart eines unter den Reaktionsbedingungen inerten Lösungsmittels, wie z.B. Toluol, Xylol oder Chloroform, in einem Temperaturbereich von 0 ° bis 100 °C durchgeführt werden. Die freien Basen werden aus den dabei anfallenden Hydrochloriden nach an sich üblichen Methoden, z.B. durch Umsetzung mit Natriumcarbonat oder Natriumhydrogencarbonat, isoliert.

Das Ausgangsmaterial der Formel III wird aus Verbindungen der Formel II durch Veresterung nach an sich üblichen Methoden mit den entsprechenden Alkoholen der Formel $R^3$-OH erhalten, wobei $R^3$ die in Formel I angegebene Bedeutung hat.

Die Verfahrensvariante C1) wird zweckmäßigerweise so durchgeführt, daß man das Ausgangsmaterial der Formel IV mit einer Mischung aus einem Alkalimetallhydrid wie Natriumhydrid und einem Alkohol der Formel $R^3$-OH, wobei $R^3$ die in Formel I angegebene Bedeutung hat, jedoch nicht Wasserstoff, im Überschuß 0,5 bis 5 Stunden bei einer Temperatur im Bereich von Raumtemperatur bis zur Siedetemperatur des Reaktionsgemisches umsetzt. Der eingesetzte Alkohol dient bei dieser Umsetzung sowohl als Reaktant als auch als Lösungsmittel. Es können jedoch auch Lösungsmittel wie z.B. Tetrahydrofuran, Dioxan oder andere nicht protische Lösungsmittel eingesetzt werden.

Die Verfahrensvariante C2) wird zweckmäßigerweise so durchgeführt, daß man das Ausgangsmaterial der Formel IV mit einem primären oder sekundären Amin der Formel $HNR^4R^5$, wobei $R^4$ und $R^5$ die in der Formel I angegebene Bedeutung haben, in einem Temperaturbereich von 20 ° bis 100 °C, gegebenenfalls unter Verwendung eines Lösungsmittels wie z.B. Dimethylformamid, Tetrahydrofuran oder Dioxan zur Reaktion bringt.

Die Verfahrensvariante C3) wird zweckmäßigerweise so durchgeführt, daß man das Ausgangsmaterial der Formel IV mit Natriumborhydrid als Reduktionsmittel in einem Temperaturbereich von -10 °C bis 30 °C in einem geeigneten Lösungsmittel oder Lösungsmittelgemisch wie z.B. Ethanol/Tetrahydrofuran zur Reaktion bringt.

Das Ausgangsmaterial der Formel IV läßt sich aus Verbindungen der allgemeinen Formel I, in der A = COOH und B = Wasserstoff bedeuten, durch Umsetzung mit Dicyclohexylcarbodiimid (DCCI) darstellen. Die Umsetzung erfolgt in einem inerten Lösungsmittel unter Verwendung einer äquimolaren Menge des Carbodiimids in einem Temperaturbereich von 20 ° bis 60 °C. Inerte Lösungsmittel für diese Reaktion sind beispielsweise halogenierte Kohlenwasserstoffe wie Chloroform und Methylenchlorid sowie cyclische Ether wie Dioxan und Tetrahydrofuran.

Zur Herstellung von Verbindungen der Formel I, bei denen der Rest $R^3$ im Substituenten A ein Kation bedeutet, wird als Ausgangsmaterial eine Verbindung der Formel I eingesetzt, bei der $R^3$ für Wasserstoff steht. Dieses wird in einem geeigneten Lösungsmittel mit einer Base als Salzbildner wie z.B. Alkalimetall- oder Erdalkalimetallhydroxid, Ammoniumhydroxid oder einem Alkylamin wie z.B. Isopropylamin, zweckmäßigerweise bei Raumtemperatur umgesetzt. Geeignete Lösungsmittel für diese Reaktion sind beispielsweise Ether wie Tetrahydrofuran und Dioxan.

Zur Herstellung von Verbindungen der Formel I, in der B für $COR^6$ oder $SO_2R^7$ und A für $COOR^3$ steht, und X, D, E, F, G, $R^1$, $R^2$ und $R^3$ die in Formel I angegebene Bedeutung haben, mit der Ausnahme, daß X und Y nicht für Alkylamino, Hydroxy oder Hydroxy-alkyl stehen können, werden die nach den Verfahrensvarianten B) oder C) synthetisierten Verbindungen der Formel I mit einer überschüssigen Menge eines Acylhalogenids der Formel $R^6$-CO-Cl, Acylanhydrids der Formel $R^6$-CO-O-CO-$R^6$ oder Sulfonylhalogenids der Formel $ClSO_2R^7$, gegebenenfalls in Gegenwart eines Lösungsmittels, wie Pyridin oder Toluol, bei einer Temperatur zwischen 50 und 130 °C umgesetzt.

Die nach den oben genannten Verfahrensvarianten hergestellten erfindungsgemäßen Verbindungen können nach üblichen Verfahren aus dem Reaktionsgemisch isoliert werden, beispielsweise indem man das Reaktionsgemisch in Eiswasser gibt und das Produkt durch Extraktion mit einem geeigneten Lösungsmittel oder durch Filtration abtrennt.

Die erfindungsgemäßen Verbindungen stellen in der Regel kristalline oder zähflüssige Substanzen dar, die zum Teil gut löslich in halogenierten Kohlenwasserstoffen wie Chloroform, Sulfoxiden wie Dimethylsulfoxid oder Estern wie Essigester sind.

Die erfindungsgemäßen Verbindungen zeigen sowohl eine große herbizide Breitenwirkung bei niedrigen Aufwandmengen gegen landwirtschaftlich wichtige monokotyle sowie dikotyle, annuelle und perennierende Unkräuter als auch eine hohe Selektivität in Hauptkulturen bei Anwendung im Vorsaat-, Vorauflauf-und Nachauflaufverfahren.

Werden die erfindungsgemäßen Verbindungen vor dem Keimen auf die Erdoberfläche appliziert, so wird das Auflaufen der Keimlinge nicht vollständig verhindert. Die Unkräuter wachsen bis zum Keimblattstadium heran, stellen jedoch dann ihr Waschstum ein und sterben schließlich ab.

Die erfindungsgemäßen Verbindungen können z.B. gegen Species aus den folgenden Pflanzengattungen verwendet werden:

Dikotyle Unkräuter der Gattungen: Abutilon, Chrysanthemum, Brassica, Helianthus, Mentha, Sinapis, Lepidium, Galium, Stellaria, Anthemis, Chenopodium, Atriplex, Senecio, Portulaca, Ipomoea, Matricaria, Galinsoga, Urtica, Amaranthus, Convolvulus, Polygonum, Sesbania, Ambrosia, Cirsium, Carduus, Sonchus, Solanum, Rorippa, Lamium, Veronica, Datura, Viola, Centaurea und Galeopsis.

Monokotyle Unkräuter der Gattungen: Avena, Alopecurus, Echinochloa, Setaria, Panicum, Digitaria, Poa, Eleusine, Brachiaria, Lolium, Bromus, Cyperus, Agropyron, Sagittaria, Cynodon, Monochoria, Fimbristylis, Eleocharis, Ischaemum und Apera.

Die Verbindungen können in landwirtschaftlichen Hauptkulturen wie Baumwolle, Soja, Reis, Mais, Weizen, Gerste, Hafer, Sorghum und Zuckerrohr angewendet werden.

Die Verwendung der erfindungsgemäßen Verbindungen ist jedoch nicht allein auf die genannten Unkrautgattungen und Kulturpflanzen beschränkt, sondern erstreckt sich in gleicher Weise auch auf andere Pflanzen.

Die erfindungsgemäßen Verbindungen eignen sich in Abhängigkeit von der Konzentration zur Totalunkrautbekämpfung z.B. auf Industrie-und Gleisanlagen und auf Wegen und Plätzen. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht-und Hopfenanlagen eingesetzt werden.

Darüberhinaus weisen die erfindungsgemäßen Verbindungen in entsprechend niederen Aufwandmengen wachstumsregulatorische Eigenschaften bei Kulturpflanzen auf, indem sie regulierend in den pflanzeneigenen Stoffwechsel eingreifen, ohne die Pflanzen abzutöten, so daß sie z.B. verwendet werden können zur generellen Steuerung und Hemmung von unerwünschtem vegetativem Wachstum und zur Verhinderung der Lagerung.

Die in der Praxis angewandten Aufwandmengen können in einem größeren Bereich schwanken. Sie hängen im wesentlichen von der Art des gewünschten Effekts ab. Im allgemeinen liegen die Aufwandmengen zwischen 0,01 und 5 kg Wirkstoff pro Hektar Bodenfläche, vorzugsweise zwischen 0,1 und 0,5 kg pro ha.

Je nach Ziel kann die Anwendung im Vor-und Nachauflauf erfolgen. Die Verbindungen können auch in den Boden eingearbeitet werden.

Sofern eine Verbreiterung des Wirkungsspektrums oder Wirkungssteigerungen beabsichtigt sind, können auch andere Herbizide zugesetzt werden. Beispielsweise eignen sich als herbizid wirksame Mischungspartner Wirkstoffe, die in Weed Abstracts, Vol: 34, No. 3, 1985, unter dem Titel "List of common names and abbreviations employed for currently used herbicides and plant growth regulators in Weed Abstracts" aufgeführt sind.

Außerdem können auch nicht phytotoxische Mittel angewendet werden, die mit Herbiziden und/oder Wuchsregulatoren eine synergistische Wirkungssteigerung ergeben können, wie unter anderem Netzmittel, Emulgatoren, Lösungsmittel und ölige Zusätze.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phsophatidylglycerol.

Zweckmäßigerweise werden die erfindungsgemäßen Wirkstoffe in Form von Zubereitungen wie Pulvern, Streumitteln, Granulaten oder Lösungen unter Zusatz von flüssigen und/oder festen Trägerstoffen beziehungsweise Verdünnungsmitteln und gegebenenfalls Netz-und/oder Haftmitteln angewandt.

Geeignete flüssige Trägerstoffe sind zum Beispiel Wasser, Methanol, Ethanol, Dimethylformamid oder Dimethylsulfoxid. Ais feste Trägerstoffe eignen sich Mineralerden wie zum Beispiel Tonsil, Silicagel, Talkum, oder Kaolin.

An oberflächenaktiven Stoffen sind zu nennen: Kationische, anionische und nicht-ionische Tenside wie zum Beispiel Calciumligninsulfonat, Polyethylenalkylphenylether, Naphthalinsulfonsäuren und deren Salze, Formaldehydkondensate, Fettalkoholsulfate sowie substituierte Benzolsulfonsäuren und deren Salze.

Der Anteil des oder der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozent flüssige oder feste Trägerstoffe sowie gegebenenfalls bis zu 40 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 1000 l/ha.

Eine Anwendung der Mittel in sogenannten "Low-Volume" oder "Ultra-Low-Volume-Verfahren" ist ebenso möglich wie ihre Applikation in Form von sogenannten Mikrogranulaten.

Zur Herstellung der Zubereitungen werden zum Beispiel die folgenden Bestandteile eingesetzt:

A. SPRITZPULVER

a) 40 Gewichtsprozent Wirkstoff
25 Gewichtsprozent Tonmineralien
20 Gewichtsprozent Kieselsäure
15 Gewichtsprozent oberflächenaktive Stoffe auf der Basis einer Mischung des Calciumsalzes der Ligninsulfonsäure mit Alkylphenolpolyglycolethern

b) 25 Gewichtsprozent Wirkstoff
60 Gewichtsprozent Kaolin
10 Gewichtsprozent Kieselsäure
5 Gewichtsprozent oberflächenaktive Stoffe auf der Basis des Natriumsalzes des N-Methyl-N-oleyl-taurins und des Calciumsalzes der Ligninsulfonsäure

B. PASTE

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

C. EMULSIONSKONZENTRAT

25 Gewichtsprozent Wirkstoff
15 Gewichtsprozent Cyclohexanon
55 Gewichtsprozent Xylol
5 Gewichtsprozent Mischung von Nonylphenylpolyoxyethylen und Calciumdodecylbenzolsulfonat.

Die folgenden Beispiele erläutern die Herstellung der erfindungsgemäßen Verbindungen:

Beispiel 1 (Verfahrensvariante A)

2-(4-Isopropyl-4-methyl-5-oxo-2-imidazolin-2-yl)-1,8-naphthyridin-3-carbonsäure

5,20 g (0,026 mol) 1,8-Naphthyridin-2,3-dicarbonsäureanhydrid werden in 150 ml Acetonitril suspendiert, mit 5,12 g (0,039 mol) 2-Methylvalin-amid versetzt und über Nacht bei Raumtemperatur gerührt. Die entstandene Lösung wird bis zur Trockne eingeengt, die gebildete 2-(N-1-Carbamoyl-1,2-dimethylpropyl)-1,8-naphthyridin-3-carbonsäure mit einer Lösung aus 14,4 g (0,36 mol) Natriumhydroxid in 144 ml Wasser versetzt und 2 Stunden bei 80 °C gerührt. Bei Raumtemperatur wird mit konzentrierter Salzsäure bis auf pH 3 angesäuert und das Produkt mit Methylenchlorid extrahiert. Die Extrakte werden über $MgSO_4$ getrocknet, eingeengt und das Endprodukt kristallin erhalten.

Ausbeute: 3,83 g (47,17 % d. Th.)

Fp.: 219 °C (Zersetzung)

Das Ausgangsmaterial wurde wie folgt hergestellt:

1,8-Naphthyridin-2,3-dicarbonsäurediethylester

Eine Mischung aus 5,65 g (0,03 mol) Oxalessigester, 3,11 g (0,025 mol) 2-Amino-3-formylpyridin, 5 ml Piperidin und 20 ml abs. Ethanol werden mit Molekularsieb (3 Å) versetzt, eine Stunde bei Raumtemperatur gerührt und anschließend 12 Stunden bei 60 °C gerührt. Anschließend wird bis zur Trockne eingeengt, der Rückstand in Ethanol aufge nommen, filtriert und wiederum bis zur Trockne eingeengt. Der Rückstand wird durch Säulenchromatographie (Kieselgel-Hexan/Essigester 1:2) gereinigt.

Ausbeute: 1,18 g (14,34 % d. Th.)

Fp.: 87-88 °C

1,8-Naphthyridin-2,3-dicarbonsäure

Eine Mischung aus 13,18 g (0,048 mol) 1,8-Naphthyridin-2,3-dicarbonsäure-diethylester, 6,0 g (0,15 mol) Natriumhydroxid und 70 ml Wasser werden 20 Stunden bei 60 °C gerührt, anschließend mit Wasser verdünnt, unter Eiskühlung mit konzentrierter Salzsäure angesäuert und der ausgefallene Niederschlag abfiltriert.

Ausbeute: 7,68 g (73,34 % d. Th.)

Fp.: >250 °C

1,8-Naphthyridin-2,3-dicarbonsäureanhydrid

0,64 g (0,003 mol) 1,8-Naphthyridin-2,3-dicarbonsäure und 6 ml Acetanhydrid werden 3 Stunden bei einer Badtemperatur von 140 °C erwärmt. Anschließend wird zur Trockne eingeengt und das Produkt sofort in die nächste Stufe eingesetzt.

Ausbeute: 0,58 g (99 % d. Th.)

Beispiel 2 (Verfahrensvariante C)

2-(4-Isopropyl-4-methyl-5-oxo-2-imidazolin-2-yl)-1,8-naphthyridin-3-carbonsäuremethylester

Eine Mischung aus 1,23 g (0,0042 mol) 2-Isopropyl-2-methyl-5H-imidazo-[1',2':1,2]pyrrolo[3,4-b]-[1,8]-naphthyridin 3(2H),5-dion, 50 mg Natriumhydrid und 12 ml Methanol werden bei Raumtemperatur über Nacht gerührt. Anschließend wird das Lösungsmittel abdestilliert und der Rückstand durch Säulenchromatographie (Kieselgel-Ether) gereinigt.

Ausbeute: 0,86 g (62,75 % d. Th.)

Fp.: 227-228 °C (aus Isopropylether)

Das Ausgangsmaterial wurde wie folgt hergestellt:

2-Isopropyl-2-methyl-5H-imidazo[1',2':1,2]pyrrolo[3,4-b]-[1,8]-naphthyridin-3(2H),5-dion

1,83 g (0,006 mol) 2-(4-Isopropyl-4-methyl-5-oxo-2-imidazolin-2-yl)-1,8-naphthyridin-3-carbonsäure und 1,34 g (0,006 mol) N,N-Dicyclohexylcarbodiimid werden in 150 ml Methylenchlorid gelöst, 4 Stunden bei Raumtemperatur gerührt und über Nacht stehengelassen. Das Reaktionsgemisch wird filtriert und das Filtrat anschließend eingeengt.

Ausbeute: 1,23 g (70,84 % d. Th.)

Fp.: >250 °C

Beispiel 3

2-(4-Isopropyl-4-methyl-5-oxo-2-imidazolin-2-yl)-1,8-naphthyridin-3-carbonsäure, Isopropylammoniumsalz

Zu einer Lösung aus 1,35 g (0,0043 mol) 2-(4-Isopropyl-4-methyl-5-oxo-2-imidazolin-2-yl)-1,8-naphthylridin-3-carbonsäure in 100 ml Tetrahydrofuran werden 0,25 g (0,0043 mol) Isopropylamin gegeben, der gebildete Niederschlag abgesaugt und getrocknet.

Ausbeute: 1,16 g (72,63 % d.Th.)

Fp.: 230 °C

Analog zu diesen Beispielen wurden auch die in der folgenden Tabelle aufgeführten Verbindungen hergestellt:

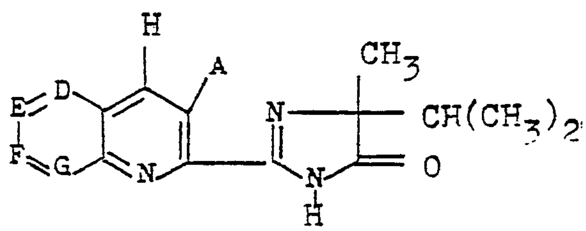

| Beispiel Nr. | D | E | F | G | A | Fp °C |
|---|---|---|---|---|---|---|
| 4 | CH | N | CH | CH | COOH | 261 (Z) |
| 5 | CH | N | CH | CH | COOH X $NH_3$ | 151 (Z) |
| 6 | CH | N | CH | CH | $COOCH_3$ | 203 (Z) |
| 7 | N | CH | CH | CH | COOH | 229 (Z) |
| 8 | N | CH | CH | CH | COOH X $NH_3$ | 226 (Z) |

Die folgenden Beispiele dienen zur Erläuterung der Anwendungsmöglichkeiten der erfindungsgemäßen Verbindungen, die in Form der obengenannten Zubereitungen erfolgte.

Beispiel 4

Im Gewächshaus wurden die erfindungsgemäßen Verbindungen in einer Aufwandmenge von 3 kg Wirkstoff/ha emulgiert in 500 l Wasser/ha auf Brassica spp. und Solanum spp. als Testpflanzen im Vorauflauf-und Nachauflauf-Verfahren gespritzt. 3 Wochen nach der Behandlung wurde das Behandlungsergebnis bonitiert, wobei folgendes Schema verwendet wurde:

0 = keine Wirkung

1 = geringe Wirkung oder schwache Inhibition des Pflanzenwuchses
2 = mittlere Wirkung oder Inhibition des Pflanzenwuchses
3 = totale Wuchsinhibition
4 = totale Vernichtung der Pflanzen

Es ergab sich, daß die Verbindungen der Beispiele 1 bis 8 in diesem Test eine 100 %ige Vernichtung (=4) der verwendeten Pflanzen bewirkten.

Beispiel 5

Im Gewächshaus wurden die in der Tabelle 1 aufgeführten Pflanzen vor dem Auflaufen mit den erfindungsgemäßen Verbindungen in einer Aufwandmenge von 1 kg Wirkstoff/ha behandelt. Die Verbindungen wurden zu diesem Zweck als Suspensionen bzw. Emulsionen mit 500 l Wasser/ha gleichmäßig über die Pflanzen versprüht. 3 Wochen nach der Behandlung zeigten die erfindungsgemäßen Verbindungen eine Selektivität in Sorghum, Weizen, Soja und Mais bei ausgezeichneter Wirkung gegen das Unkraut. Das Vergleichsmittel Scepter war weniger selektiv.

0 = keine Wirkung
1 = geringe Wirkung oder schwache Inhibition des Pflanzenwuchses
2 = mittlere Wirkung oder Inhibition des Pflanzenwuchses
3 = totale Wuchsinhibition
4 = totale Vernichtung der Pflanzen

Tabelle 1

| Verbindung gemäß Beispiel | Sorghum sativum | Glycine max. | Zea mays | Triticum aestivum | Brassica sp. | Solanum sp. | Medicago sp. | Helianthus sp. | Stellaria sp. | Abutilon sp. | Matricaria sp. | Chrysanthemum sp. | Setaria sp. |
|---|---|---|---|---|---|---|---|---|---|---|---|---|---|
| 1 | 0 | 0 | 0 | 0 | 4 | 4 | 4 | 4 | 4 | 4 | 4 | 4 | 4 |
| 3 | 0 | 0 | 0 | 0 | 4 | 4 | 4 | 4 | 4 | 4 | 4 | 4 | 4 |
| Vergleichsmittel Scepter *) | 4 | 0 | 4 | 4 | 4 | 4 | 4 | 4 | 4 | 4 | 4 | 4 | 4 |

*) 2-(4-Isopropyl-4-methyl-5-oxo-2-imidazolin-2-yl)-chinolin-3-carbonsäure

Beispiel 6

Im Gewächshaus wurden die in der Tabelle 2 aufgeführten Pflanzen nach dem Auflaufen mit den erfingungsgemäßen Verbindungen in einer Aufwandmenge von 1 kg Wirkstoff/ha behandelt. Die Verbindungen wurden zu diesem Zweck als Suspensionen bzw. Emulsionen mit 500 l Wasser/ha gleichmäßig über die Pflanzen versprüht. 3 Wochen nach der Behandlung zeigten die erfindungsgemäßen Verbindungen eine Selektivität in Sorghum, Soja, Weizen und Mais bei ausgezeichneter Wirkung gegen das Unkraut. Das verwendete Vergleichsmittel war weniger selektiv.

0 = keine Wirkung
1 = geringe Wirkung oder schwache Inhibition des Pflanzenwuchses
2 = mittlere Wirkung oder Inhibition des Pflanzenwuchses
3 = totale Wuchsinhibition
4 = totale Vernichtung der Pflanzen

Tabelle 2

| Verbindung gemäß Beispiel | Sorghum sativum | Zea mays | Triticum aestivum | Glycine max. | Brassica sp. | Solanum sp. | Medicago sp. | Helianthus sp. | Stellaria sp. | Abutilon sp. | Matricaria sp. | Chrysanthemum sp. | Setaria sp. |
|---|---|---|---|---|---|---|---|---|---|---|---|---|---|
| 1 | 0 | 0 | 0 | 0 | 4 | 4 | 4 | 4 | 4 | 4 | 4 | 4 | 4 |
| 3 | 0 | 0 | 0 | 0 | 4 | 4 | 4 | 4 | 4 | 4 | 4 | 4 | 4 |
| Vergleichs-mittel Scepter | 4 | 4 | 4 | 0 | 4 | 4 | 4 | 4 | 4 | 4 | 4 | 4 | 4 |

**Ansprüche**

1. Naphthyridin-Derivate der allgemeinen Formel I

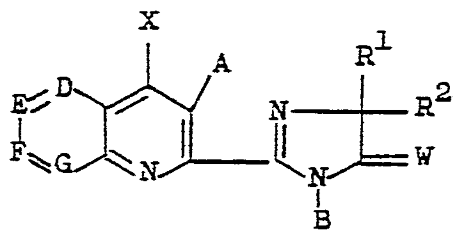

(I),

in der eines der Symoble D, E, F oder G ein Stickstoffatom und die anderen Symbole ein mit Y substituiertes Kohlenstoff-Atom bedeuten, wobei

Y für Wasserstoff, Halogen, Amino, Nitro, Cyano, $C_{3-8}$-Cycloalkyl, $C_{3-8}$ -Cycloalkenyl, $C_{3-8}$-Cycloalkyloxy, $C_{3-8}$-Cycloalkenyloxy, $C_{1-8}$-Alkoxy, $C_{1-8}$-Alkoxycarbonyl, $C_{1-8}$-Alkylthio, $C_{3-8}$-Cycloalkylthio, Benzylthio, Tetrahydrofurfuryloxy; für freies oder mit Halogen, $C_{1-4}$-Alkyl oder $C_{1-4}$-Alkoxy substituiertes Phenyl; für freies oder mit $C_{1-4}$-Alkyl, Halogenalkyl oder Nitro substituiertes Phenoxy; für $C_{1-4}$-Alkylamino, Di-$C_{1-4}$-alkylamino, $C_{3-6}$-Cycloalkylamino; oder für einen ein-oder mehrfach mit Halogen, Cyano, Nitro, Amino oder $C_{1-4}$-Alkoxy substituierten $C_{1-8}$-Alkyl-, $C_{3-8}$-Alkenyl-oder $C_{3-8}$-Alkinylrest steht,

X für Wasserstoff, Nitro, $C_{1-3}$-Alkoxy, $C_{1-3}$-Halogenalkyl und $C_{1-3}$-Alkyl steht,

$R^1$ für $C_{1-4}$-Alkyl steht,

$R^2$ für $C_{1-6}$-Alkyl oder $C_{3-6}$-Cycloalkyl steht, wobei

$R^1$ und $R^2$ gemeinsam einen gegebenenfalls mit Methyl substituierten $C_{3-6}$-Cycloalkylrest bedeuten können,

W für O oder S steht,

A für $CH_2OH$, $COOR^3$ oder $CONR^4R^5$ steht, wobei $R^3$ Wasserstoff, $C_{1-12}$-Alkyl, das durch ein oder mehrere O oder S unterbrochen sein kann und substituiert sein kann mit $C_{1-3}$-Alkoxy, Halogen, Hydroxy, $C_{3-6}$-Cycloalkyl, Benzyl, Furyl, Tetrahydrofuryl, Phenyl, Halogenphenyl, $C_{1-4}$-Alkylphenyl, $C_{1-4}$-Alkoxyphenyl, Nitrophenyl, Cyano, Carboxyl, $C_{1-4}$-Alkoxycarbonyl oder $C_{1-4}$-Alkylthio; $C_{3-8}$-Alkenyl, das mit $C_{1-3}$-Alkoxy, Phenyl oder Halogen substituiert sein kann; $C_{3-8}$-Alkinyl, das mit $C_{1-3}$-Alkoxy, Phenyl oder Halogen substituiert sein kann; $C_{3-6}$-Cycloalkyl, gegebenenfalls substituiert mit Methyl; oder ein Kation aus der Gruppe der Alkalimetalle, Erdalkalimetalle, Mangan, Kupfer, Eisen, Zink, Ammonium und organischen Ammoniumverbindungen bedeutet, und

$R^4$ und $R^5$ unabhängig voneinander für Wasserstoff, Hydroxy und $C_{1-4}$-Alkyl stehen, und falls $R^4$ Wasserstoff bedeutet, $R^5$ auch für $NH_2$, $N(CH_3)_2$, $NHCOCH_3$ und NH-Phenyl steht,

und

B für Wasserstoff steht und falls A $COOR^3$ ($R^3 \neq$ Wasserstoff) bedeutet, B auch $COR^6$ oder $SO_2R^7$ bedeutet, wobei $R^6$ für $C_{1-8}$-Alkyl, $C_{1-6}$-Halogenalkyl oder Phenyl steht, das mit Halogen, Nitro, Methyl oder Methoxy substituiert sein kann, und $R^7$ für $C_{1-6}$ -Alkyl, $CF_3$, $CCl_3$ oder für ein gegebenenfalls mit Halogen, Nitro oder $C_{1-6}$-Alkyl substituiertes Phenyl steht.

2. Verfahren zur Herstellung von Verbindungen der Formel I, dadurch gekennzeichnet, daß man

A) Verbindungen der allgemeinen Formel II

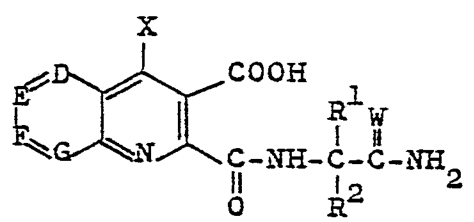

(II),

in der X, D, E, F, G, $R^1$, $R^2$ und W die in Formel I angegebene Bedeutung haben, alkalisch cyclisiert, wobei Verbindungen der Formel I erhalten werden, in der A = COOH und B = Wasserstoff bedeuten;

B) Verbindungen der allgemeinen Formel III

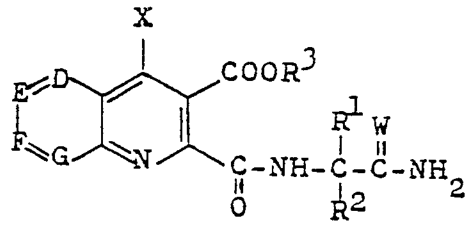

(III),

in der X, D, E, F, G, $R^1$, $R^2$, $R^3$ (jedoch nicht Wasserstoff) und W die in Formel I angegebene Bedeutung haben, mit einem Gemisch aus Phosphoroxichlorid/Phosphorpentachlorid umsetzt, wobei Verbindungen der Formel I erhalten werden, in der A = $COOR^3$ ($R^3 \neq H$) und B = Wasserstoff bedeuten;

C) Verbindungen der allgemeinen Formel IV

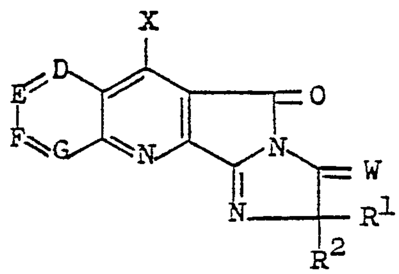

(IV),

in der X, D, E, F, G, W, $R^1$ und $R^2$ die in Formel I angegebene Bedeutung haben,

C1) entweder mit einem Alkohol der Formel $R^3$-OH, wobei $R^3$ die in Formel I angegebene Bedeutung hat, jedoch nicht Wasserstoff, und dem entsprechenden Alkalimetallalkoxid, gegebenenfalls in Gegenwart eines Lösungsmittels umsetzt, oder

C2) mit einem Amin der Formel $HNR^4R^5$, wobei $R^4$ und $R^5$ die in Formel I angegebene Bedeutung haben, gegebenenfalls unter Verwendung eines Lösungsmittels umsetzt,

oder

C3) mit einem Reduktionsmittel, vorzugsweise Natriumborhydrid, zur Reaktion bringt,

wobei Verbindungen der allgemeinen Formel I erhalten werden, in der A = $COOR^3$ ($R^3 \neq H$), $CONR^4R^5$ oder $CH_2OH$ und B = Wasserstoff bedeuten;

und gegebenenfalls eine Verbindung der Formel I, in der A für COOH steht, mit 1 Äquivalent einer Base als Salzbildner umsetzt, wobei Verbindungen der Formel I erhalten werden, in denen A für $COOR^3$ steht und $R^3$ ein salzbildendes Kation bedeutet,

oder

gegebenenfalls eine Verbindung der Formel I, in der A für $CO_2R^3$ ($R^3 \neq H$) steht, mit einem Acylanhydrid oder Sulfonylhalogenid umsetzt, wobei Verbindungen der Formel I erhalten werden, in denen B für $COR^6$ oder $SO_2R^7$ steht.

3. Mittel mit herbizider Wirkung, gekennzeichnet durch einen Gehalt an mindestens einer Verbindung gemäß Anspruch 1,

4. Mittel gemäß Anspruch 3 in Mischung mit Träger-und/oder Hilfsstoffen.

5. Verwendung der Verbindungen gemäß Anspruch 1 zur Bekämpfung dikotyler und monokotyler Pflanzenarten in Nutzpflanzkulturen.